# EUROPÄISCHE PATENTSCHRIFT

(11) **EP 0 467 252 B1**
(45) Veröffentlichungstag und Bekanntmachung des Hinweises auf die Patenterteilung: **03.06.1998**
(21) Anmeldenummer: 91111725.7
(22) Anmeldetag: 13.07.1991
(51) Int. Cl.: C07D 239/42, C07D 239/52

(54) **Verfahren zur Herstellung von N-Alkylsulfonylaminosulfonylharnstoffen**
Process for the preparation of N-alkylsulfonylaminosulfonyl ureas
Procédé de préparation de N-alkylsulfonylaminosulfonyl urées

(30) Priorität: 19.07.1990 DE 4022983
(43) Veröffentlichungstag der Anmeldung: 22.01.1992
(73) Patentinhaber: Hoechst Schering AgrEvo GmbH, 13342 Berlin (DE)
(72) Erfinder: Lachhein, Stephen, Dr., W-6238 Hofheim a.Ts. (DE); Willms, Lothar, Dr., W-5416 Hillscheid (DE)

(56) Entgegenhaltungen:
- EP-A- 0 131 258
- EP-A- 0 342 456
- EP-A- 0 353 641

## Beschreibung

Gegenstand der vorliegenden Erfindung ist ein Verfahren zur Herstellung der Verbindungen der Formel I, 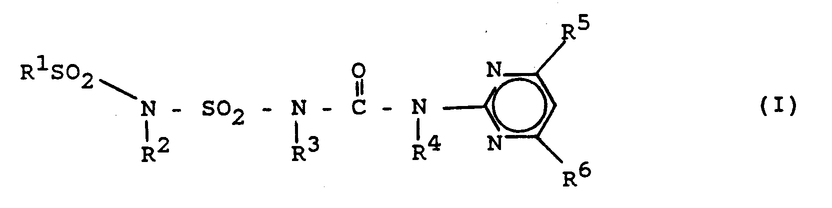 worin
- R¹: (C₁-C₆)-Alkyl, (C₂-C₆)-Alkenyl oder (C₂-C₆)-Alkinyl, wobei jeder der genannten 3 Reste unsubstituiert oder ein- oder mehrfach durch Reste aus der Gruppe Halogen, (C₁-C₄)-Alkoxy und [(C₁-C₄)-Alkoxy]-carbonyl substituiert ist,
- R²: Wasserstoff, (C₁-C₆)-Alkyl, (C₂-C₆)-Alkenyl, (C₂-C₆)-Alkinyl oder (C₃-C₆)-Cycloalkyl,
- R³, R⁴: unabhängig voneinander Wasserstoff oder (C₁-C₄)-Alkyl,
- R⁵, R⁶: unabhängig voneinander Wasserstoff, (C₁-C₄)-Alkyl oder (C₁-C₄)-Alkoxy, wobei die 2 letztgenannten Reste unsubstituiert oder ein- oder mehrfach durch Reste aus der Gruppe Halogen, Alkoxy und Alkylthio substituiert sind, Halogen, (C₁-C₄)-Alkylthio, (C₁-C₄)-Alkylamino oder (C₁-C₄)-Dialkylamino
bedeuten, sowie, falls R², R³ Wasserstoff bedeuten, deren physiologisch verträglichen Salze mit Basen.

Verbindungen der Formel I sind bekannt und werden als Pflanzenschutzmittel mit herbizider Wirkung eingesetzt; siehe z.B. EP-A 0 131 258.

Dort sind auch schon einige Verfahren zitiert oder beschrieben, nach denen Verbindungen der Formel I hergestellt werden können.

Nachteilig bei den bekannten Verfahren sind die relativ niedrigen Ausbeuten von höchstens 65-70 %. Bedingt durch diese niedrigen Ausbeuten fallen erhebliche Mengen an
Verunreinigungen und Nebenprodukten an. Diese beschriebenen Verfahren sind aus ökologischer als auch aus ökonomischer Sicht nicht in einem großtechnischen Maßstab durchführbar, da hierbei unvertretbar große Mengen an Nebenprodukten und Abfällen entstehen würden, die z.B. durch Verbrennung aufwendig entsorgt werden müßten. Außerdem tritt bei solch niedriger Ausbeute ein drastischer Verlust an eingesetzten Ausgangsmaterialien ein.

Es wurde nun ein neues Verfahren gefunden, nach dem die Verbindungen der Formel I in überraschend hoher Ausbeute und Reinheit herstellbar sind.

Das erfindungsgemäße Verfahren zeichnet sich dadurch aus, daß man Verbindungen der Formel II,

R¹ - SO₂ - NR² - SO₂ - NR³ - CO - OR⁷ (II)

worin
- R¹, R² und R³: wie oben definiert sind und
- R⁷: Alkyl, Haloalkyl, gegebenenfalls substituiertes Phenyl bedeuten,
mit Verbindungen der Formel III, 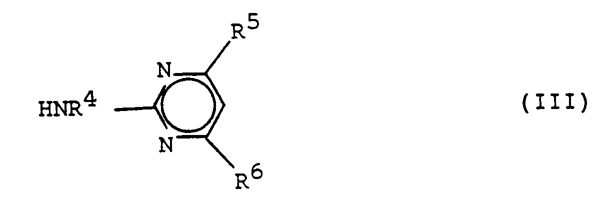 worin
- R⁴, R⁵ und R⁶: wie oben definiert sind,
in einem inerten organischen Lösungsmittel zu den Verbindungen der Formel I umsetzt.

In den genannten Formeln und im folgenden können Alkyl-, Alkoxy-, Haloalkyl-, Alkylamino- und Alkylthioreste sowie die entsprechenden ungesättigten und/oder substituierten Reste, wenn nicht anders angegeben, bezüglich der Kohlenstoffkette jeweils geradkettig oder verzweigt sein; Alkylreste, auch in den zusammengesetzten Bedeutungen wie Alkoxy, Haloalkyl usw., bedeuten beispielsweise Methyl, Ethyl, n- oder i-Propyl, n-, i-, t- oder 2-Butyl; Alkenylund Alkinylreste haben die Bedeutung der den Alkylresten entsprechenden möglichen ungesättigten Reste, wie 2-Propenyl, 2- oder 3-Butenyl, 2-Propinyl, 2- oder 3-Butinyl; gegebenenfalls substituiertes Phenyl bedeutet beispielsweise Phenyl, das unsubstituiert oder durch ein oder mehrere, vorzugsweise 1 bis 3 Reste aus der Gruppe Halogen, (C₁-C₄)-Alkyl, (C₁-C₄)-Alkoxy, (C₁-C₄)-Haloalkyl, (C₁-C₄)-Thioalkyl, (C₁-C₄-Alkoxy)-carbonyl, (C₁-C₄-Alkyl)-sulfonyl, Cyano und Nitro sustituiert ist; Halogen, auch Halo in Haloalkyl etc., bedeutet Fluor, Chlor, Brom oder Jod, vorzugsweise Fluor, Chlor oder Brom.

Unter den erfindungsgemäßen Verfahren zur Herstellung der Verbindungen der Formel I sind diejenigen bevorzugt, in denen R¹, R² unabhängig voneinander (C₁-C₃)-Alkyl oder (C₁-C₃)-Alkenyl, insbesondere (C₁-C₂)-Alkyl, R³ und R⁴ Wasserstoff, R⁵ und R⁶ unabhängig voneinander (C₁-C₂)-Alkyl oder (C₁-C₂)-Alkoxy, insbesondere Methyl oder Methoxy, bedeuten.

R⁷ ist vorzugsweise (C₁-C₄)-Alkyl, (C₁-C₄)-Haloalkyl oder Phenyl, insbesondere Ethyl oder Phenyl.

Die Ausbeuten nach dem erfindungsgemäßen Verfahren liegen in der Regel bei mindestens 95 % d.Th. und die Reinheiten der entstehenden Sulfonylharnstoffe I sind meist größer als 98 Gew.-%.

Das erfindungsgemäße Verfahren wird in inerten organischen Lösungsmitteln durchgeführt. Beispiele für Lösungsmitteltypen sind dabei aromatische, gegebenenfalls halogenierte Kohlenwasserstoffe und aprotisch polare Lösungsmittel wie Dialkylalkanoylamide, Dialkylsulfoxide, Polyalkylenglykoldialkylether, N-alkylierte cyclische Amide und Nitrile. Bevorzugt sind Lösungsmittel wie z.B. Toluol, Xylol, Chlorbenzol, Dimethylformamid, Dimethylsulfoxid, Di-, Tri- oder Tetraethylenglykoldialkylether, insbesondere -dimethyl oder -diethylether, N-Methylpyrrolidon, Acetonitril oder auch Mischungen aus zwei oder mehreren der genannten Lösungsmittel.

In der Regel wird die Verbindung der Formel II im Verhältnis zur Verbindung der Formel III äquimolar oder in geringem Überschuß eingesetzt. Bevorzugt ist ein Molverhältnis für II:III von 1:1 bis 1,2:1, insbesondere 1:1 bis 1,1:1.

Ein Vorteil des erfindungsgemäßen Verfahrens besteht darin, daß die Lösungsmittel in nahezu quantitativer Ausbeute wieder recyclisiert werden können, da die Produkte der Formel I als schwerlösliche Verbindungen aus dem Reaktionsmedium in hoher Reinheit und Ausbeute ausfallen. Die Lösungsmittel können anschließend z.B. durch Destillation gereinigt und dann wieder in den Produktionsprozeß eingeführt werden.

Die Reaktionstemperaturen liegen vorzugsweise bei 0°C bis zum Siedepunkt des eingesetzten Lösungsmittels, insbesondere bei Raumtemperatur (z.B. 20°C) bis 110°C.

Die für die Herstellung der erfindungsgemäßen Verbindungen der allgemeinen Formel I benötigten Ausgangsverbindungen der Formeln II und III lassen sich nach literaturbekannten Verfahren herstellen.

So werden die Verbindungen der Formel II analog üblichen Methoden (siehe z.B. Tietze und Eicher in "Reaktionen und Synthesen", S. 92, Thieme Verlag, Stuttgart 1981) durch Umsetzung der entsprechenden Sulfonamide IV mit Chlorameisensäureestern V erhalten,

R¹-SO₂-NR²-SO₂-NH-R³ (IV)

Cl-CO-OR⁷ (V)

die ihrerseits wiederum durch Umsetzung der entsprechenden Sulfonamide VI mit den entsprechenden Amidosulfochloriden VII

R¹-SO₂-NH-R² (VI)

Cl-SO₂-NH-R³ (VII)

nach laborüblichen Methoden (siehe z.B. "Organikum", 7. Auflage, S. 539, VEB Deutscher Verlag der Wissenschaften, Berlin 1967) in sehr hoher Ausbeute zugänglich sind.

Die Heterocyclen der Formel III sind entweder käuflich oder lassen sich nach geeigneten Labormethoden leicht herstellen; s. z.B. US 4,310,470; EP 0 024 200; US 4,299,960; M.J. Langerman, C.K. Banks, JACS 73, 3011 (1951).

Als besonders überraschend ist das erfindungsgemäße Verfahren deshalb anzusehen, weil das Ausgangsprodukt der Formel II mehrere aktivierte elektrophile und nucleophile Zentren enthält, wobei insbesondere die elektrophilen Zentren prinzipiell alle mit den nucleophilen Substanzen der Formel III reagieren könnten und so durch Fragmentierungsreaktionen eine Vielzahl von Nebenprodukten liefern könnten; vgl. Beyer, Lehrbuch der org. Chemie, 19. Auflage, S. 128, Hirzel Verlag Stuttgart, wonach Sulfonylgruppen sehr gute Abgangsgruppen darstellen.

Die genannten Nebenreaktionen treten aber erstaunlicherweise bei dem erfindungsgemäßen Verfahren praktisch nicht auf, denn nach dem erfindungsgemäßen Verfahren werden meist Ausbeuten von über 95 % d.Th. und Reinheiten von über 98 % erhalten.

Somit stellt das erfindungsgemäße Verfahren einen neuen, einfachen, auch im größeren technischen Maßstab gut reproduzierbaren und hochselektiven Prozeß zur Darstellung der Verbindungen der Formel I in nahezu quantitativen Ausbeuten dar.

Das Verfahren kann diskontinuierlich oder kontinuierlich durchgeführt werden.

Das erfindungsgemäße Verfahren wird nachfolgend an einigen Beispielen erläutert. Sofern nichts anderes angegeben ist, beziehen sich Prozentangaben auf das Gewicht.

### Beispiel 1

### 1-[(N-Methylsulfonyl-N-methyl-amino)-sulfonyl]-3-(4,6-dimethoxy-2-pyrimidyl)-harnstoff

52,0 g O-Ethyl-N-[(N-Methylsulfonyl-N-methylamino)-sulfonyl]-carbamat werden in 500 ml Chlorbenzol gelöst und bei Raumtemperatur unter Rühren mit 31,0 g 2-Amino-4,6-dimethoxypyrimidin versetzt und für 3 Stunden auf 80°C erhitzt. Nach Abkühlen auf 0°C wird der Niederschlag abfiltriert und mit 100 ml Chlorbenzol gewaschen. Man erhält 72,7 g des gewünschten Produkts mit einer Reinheit von 98,5 %, was einer Ausbeute von 97,2 % d.Th. entspricht. Der Schmelzpunkt für das Produkt liegt bei 185-186°C.

### Beispiel 2

### 1-[(N-Methylsulfonyl-N-methyl-amino)-sulfonyl]-3-(4-methoxy-6-methyl-2-pyrimidyl)-harnstoff

52,0 g O-Ethyl-N-[(N-Methylsulfonyl-N-methylamino)-sulfonyl]-carbamat werden in 500 ml Chlorbenzol gelöst, bei Raumtemperatur mit 27,8 g 2-Amino-4-methoxy-6-methyl-pyrimidin versetzt und für 5 Stunden auf 50°C erhitzt. Nach Abkühlen auf 0°C wird der Niederschlag abfiltriert. Nach Waschen mit 100 ml Chlorbenzol erhält man 68,8 g des gewünschten Produkts mit einer Reinheit von 98,9 %; das entspricht einer Ausbeute von 96,4 % d.Th. Der Schmelzpunkt des Produkts ist 118-120°C.

### Beispiel 3

### 1-[(N-Ethylsulfonyl-N-ethyl-amino)-sulfonyl]-3-(4,6-diethoxy-2-pyrimidyl)-harnstoff

65,2 g O-Phenyl-N-[(N-Ethylsulfonyl-N-methylamino)-sulfonyl]-carbamat werden in 700 ml Toluol gelöst, bei Raumtemperatur mit 36,6 g 2-Amino-4,6-diethoxypyrimidin versetzt und für 2 Stunden auf 110°C erhitzt. Nach Abkühlen auf Raumtemperatur wird der Niederschlag bei 0°C abfiltriert und mit 200 ml Toluol nachgewaschen.

Man erhält 82,6 g des gewünschten Produkts mit einer Reinheit von 98,1 %, was einer Ausbeute von 95,3 % d.Th. entspricht.
Der Schmelzpunkt des Produkts ist 174-176°C.

Analog den Beispielen 1 bis 3 können die in nachfolgender Tabelle 1 aufgeführten Verbindungen der Formel I synthetisiert werden.

**Tabelle 1**

| Bsp. Nr. | R¹ | R² | R³ | R⁴ | R⁵ | R⁶ | Smp. (°C) |
|---|---|---|---|---|---|---|---|
| 4 | CH₃ | CH₃ | H | H | CH₃ | CH₃ | 150-151 |
| 5 | CH₃ | C₃H₇ | H | H | CH₃ | CH₃ | 149-151 |
| 6 | CH₃ | C₃H₇ | H | H | OCH₃ | CH₃ | 141-143 |
| 7 | CH₃ | CH₂=CHCH₂ | H | H | CH₃ | CH₃ | 139-141 |
| 8 | CH₃ | CH₂=CHCH₂ | H | H | OCH₃ | CH₃ | 159-161 |
| 9 | CH₂Cl | CH₃ | H | H | CH₃ | CH₃ | 146-148 |
| 10 | CH₃ | C₃H₇ | H | H | OCH₃ | OCH₃ | 156-157 |
| 11 | CH₃ | CH(CH₃)₂ | H | H | OCH₃ | OCH₃ | 121-123 |
| 12 | CH₃ | CH(CH₃)₂ | H | H | Cl | OCH₃ | 153-155 |
| 13 | C₂H₅ | C₂H₅ | H | H | OCH₃ | OCH₃ | |
| 14 | CH₃ | CH₃ | CH₃ | H | OCH₃ | OC₂H₅ | |
| 15 | CH₃ | CH₃ | H | CH₃ | OCH₃ | OCH₃ | |
| 16 | CH₃ | C₂H₅ | CH₃ | CH₃ | C₂H₅ | OCH₃ | |
| 17 | C₃H₇ | CH₃ | H | H | CH₃ | CH₃ | |
| 18 | C₄H₉ | CH₃ | H | H | OCH₃ | OCH₃ | |
| 19 | CH₃ | cyclo-C₆H₁₁ | H | H | OCH₃ | OCH₃ | |
| 20 | CH₃ | CH₂-C≡CH | CH₃ | H | CH₃ | OCF₂H | |
| 21 | CH₃ | CH₂-CO₂CH₃ | H | H | CH₃ | CH₃ | |

## Patentansprüche

1. Verfahren zur Herstellung von Verbindungen der Formel I 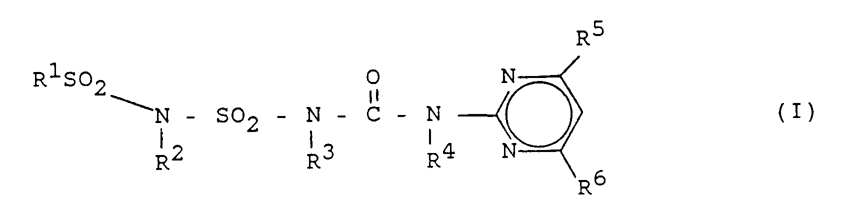 worin
R¹ (C₁-C₆)-Alkyl, (C₂-C₆)-Alkenyl oder (C₂-C₆)-Alkinyl, wobei jeder der genannten 3 Reste unsubstituiert oder ein- oder mehrfach durch Halogen, (C₁-C₄)-Alkoxy und [(C₁-C₄)-Alkoxy]-carbonyl substituiert ist,
R² Wasserstoff, (C₁-C₆)-Alkyl, (C₂-C₆)-Alkenyl, (C₂-C₆)-Alkinyl oder (C₃-C₆)-Cycloalkyl,
R³, R⁴ unabhängig voneinander Wasserstoff oder (C₁-C₄)-Alkyl,
R⁵, R⁶ unabhängig voneinander Wasserstoff, (C₁-C₄)-Alkyl oder (C₁-C₄)-Alkoxy, wobei die 2 letztgenannten Reste unsubstituiert oder ein- oder mehrfach durch Reste aus der Gruppe Halogen, Alkoxy und Alkylthio substituiert sind, Halogen, (C₁-C₄)-Alkylthio, (C₁-C₄)-Alkylamino oder (C₁-C₄)-Dialkylamino
bedeuten, sowie, falls R², R³ Wasserstoff bedeuten, deren physiologisch verträglichen Salzen mit Basen, dadurch gekennzeichnet, daß man Verbindungen der Formel II,
R¹ - SO₂ - NR² - SO₂ - NR³ - CO - OR⁷ (II)
worin
R¹, R², R³ wie oben definiert sind, und
R⁷ Alkyl, Haloalkyl oder gegebenenfalls substituiertes Phenyl bedeuten, mit Verbindungen der Formel III, 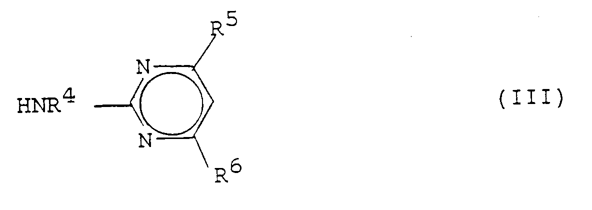 worin
R⁴, R⁵ und R⁶ wie oben definiert sind,
in einem inerten organischen Lösungsmittel zu den Verbindungen der Formel I umsetzt.

2. Verfahren nach Anspruch 1, dadurch gekennzeichnet, daß R¹, R² unabhängig voneinander (C₁-C₃)-Alkyl oder (C₁-C₃)-Alkenyl, R³ und R⁴ Wasserstoff, R⁵ und R⁶ unabhängig voneinander (C₁-C₂)-Alkyl oder (C₁-C₂)-Alkoxy bedeuten.

3. Verfahren nach Anspruch 1 oder 2, dadurch gekennzeichnet, daß R¹ (C₁-C₂)-Alkyl, R² (C₁-C₃)-Alkyl oder (C₁-C₃)-Alkenyl, R³ und R⁴ Wasserstoff, R⁵ Methyl oder Methoxy und R⁶ Methyl oder Methoxy bedeuten.

4. Verfahren nach einem oder mehreren der Ansprüche 1 bis 3, dadurch gekennzeichnet, daß R⁷ (C₁-C₄)-Alkyl, (C₁-C₄)-Haloalkyl oder Phenyl ist.

5. Verfahren nach einem oder mehreren der Ansprüche 1 bis 4, dadurch gekennzeichnet, daß man als inerte organische Lösungsmittel aromatische Kohlenwasserstoffe, halogenierte aromatische Kohlenwasserstoffe oder aprotisch polare Lösungsmittel oder Mischungen der genannten Lösungsmittel einsetzt.

6. Verfahren nach einem oder mehreren der Ansprüche 1 bis 5, dadurch gekennzeichnet, daß man es bei Reaktionstemperaturen von 0°C bis zum Siedepunkt des eingesetzten Lösungsmittels durchführt.

7. Verfahren nach Anspruch 6, dadurch gekennzeichnet, daß die Reaktionstemperatur bei Raumtemperatur bis 110°C ist.

8. Verfahren nach einem oder mehreren der Ansprüche 1 bis 7, dadurch gekennzeichnet, daß die Verbindungen II und III im Molverhältnis von 1:1 bis 1,2:1 umgesetzt werden.

9. Verfahren nach Anspruch 8, dadurch gekennzeichnet, daß das Molverhältnis 1:1 bis 1,1:1 ist.

10. Verfahren nach einem oder mehreren der Ansprüche 1 bis 9, dadurch gekennzeichnet, daß das Verfahren diskontinuierlich oder kontinuierlich durchgeführt wird.

## Claims

1. A process for preparing compounds of the formula I 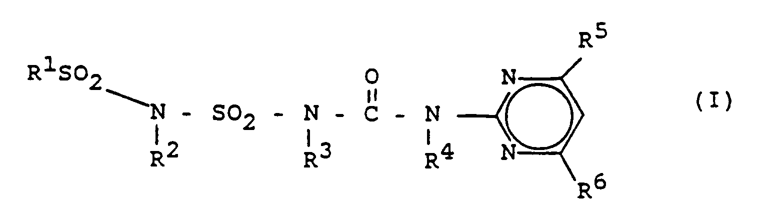 in which
R¹ is (C₁-C₆)-alkyl, (C₂-C₆)-alkenyl or (C₂-C₆)-alkynyl, each of the abovementioned three radicals being unsubstituted or mono- or polysubstituted by halogen, (C₁-C₄)-alkoxy and [(C₁-C₄)-alkoxy]-carbonyl,
R² is hydrogen, (C₁-C₆)-alkyl, (C₂-C₆)-alkenyl, (C₂-C₆)-alkynyl or (C₃-C₆)-cycloalkyl,
R³ and R⁴ independently of one another are each hydrogen or (C₁-C₄)-alkyl,
R⁵ and R⁶ independently of one another are each hydrogen, (C₁-C₄)-alkyl or (C₁-C₄)-alkoxy, the two last-mentioned radicals being unsubstituted or mono- or polysubstituted by radicals selected from the group consisting of halogen, alkoxy and alkylthio, or are halogen, (C₁-C₄)-alkylthio, (C₁-C₄)-alkylamino or (C₁-C₄)-dialkylamino
and, if R² and R³ are hydrogen, physiologically acceptable salts thereof with bases, which comprises reacting compounds of the formula II
R¹ - SO₂ - NR² - SO₂ - NR³ - CO - OR⁷ (II)
in which
R¹, R², R³ and R⁴ are each as defined above and
R⁷ is alkyl, haloalkyl or phenyl with or without substitution, with compounds of the formula III 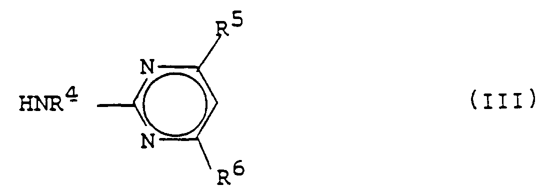 in which
R⁴, R⁵ and R⁶ are each as defined above,
in an inert organic solvent to give compounds of the formula I.

2. The process as claimed in claim 1, wherein R¹ and R² independently of one another are each (C₁-C₃)-alkyl or (C₁-C₃)-alkenyl, R³ and R⁴ are each hydrogen, R⁵ and R⁶ independently of one another are each (C₁-C₂)-alkyl or (C₁-C₂)-alkoxy.

3. The process as claimed in claim 1 or 2, wherein R¹ is (C₁-C₂)-alkyl, R² is (C₁-C₃)-alkyl or (C₁-C₃)-alkenyl, R³ and R⁴ are each hydrogen, R⁵ is methyl or methoxy and R⁶ is methyl or methoxy.

4. The process as claimed in one or more of claims 1 to 3, wherein R⁷ is (C₁-C₄)-alkyl, (C₁-C₄)-haloalkyl or phenyl.

5. The process as claimed in one or more of claims 1 to 4, wherein the inert organic solvents used are aromatic hydrocarbons, halogenated aromatic hydrocarbons or aprotic polar solvents or mixtures of the abovementioned solvents.

6. The process as claimed in one or more of claims 1 to 5, wherein the process is carried out at reaction temperatures of from 0°C to the boiling point of the solvent used.

7. The process as claimed in claim 6, wherein the reaction temperature is from room temperature to 110°C.

8. The process as claimed in one or more of claims 1 to 7, wherein the compounds II and III are reacted at a molar ratio of 1:1 to 1.2:1.

9. The process as claimed in claim 8, wherein the molar ratio is from 1:1 to 1.1:1.

10. The process as claimed in one or more of claims 1 to 9, wherein the process is carried out batchwise or continuously.

## Revendications

1. Procédé de préparation de composes de formule I : 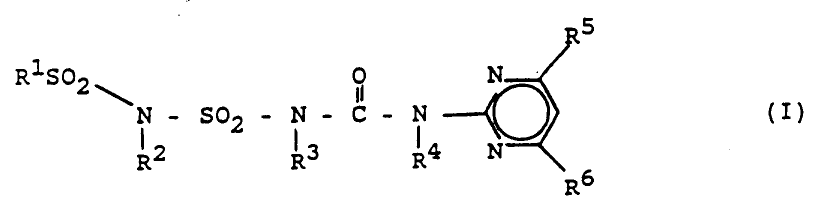 dans laquelle
R¹ représente un groupe alkyle en C₁-C₆, alcényle en C₂-C₆ ou alcynyle en C₂-C₆, chacun des 3 radicaux cités étant non substitué ou une ou plusieurs fois substitué par un atome d'halogène, un groupe alkoxy en C₁-C₄ et (alkoxy en C₁-C₄)-carbonyle,
R² représente un atome d'hydrogène, un groupe alkyle en C₁-C₆, alcényle en C₂-C₆, alcynyle en C₂-C₆ ou cycloalkyle en C₃-C₆,
R³, R⁴ représentent indépendamment l'un de l'autre un atome d'hydrogène ou un groupe alkyle en C₁-C₄,
R⁵, R⁶ représentent indépendamment l'un de l'autre un atome d'hydrogène, un groupe alkyle en C₁-C₄ ou alkoxy en C₁-C₄, les 2 derniers radicaux cités étant non substitués ou une ou plusieurs fois substitués par des résidus choisis parmi un atome d'halogène, un groupe alkoxy et alkylthio, ou représentent un atome d'halogène, un groupe alkylthio en C₁-C₄, alkylamino en C₁-C₄ ou dialkylamino en C₁-C₄,
ainsi que, dans le cas où R², R³ représentent des atomes d'hydrogène, de leurs sels physiologiquement acceptables avec des bases, caractérisé en ce que, l'on fait réagir des composés de formule II,
R¹ - SO₂ - NR² - SO₂ - NR³ - CO - OR⁷ (II)
dans laquelle
R¹, R² et R³ sont définis comme ci-dessus, et
R⁷ représente un groupe alkyle, haloalkyle, ou phényle éventuellement substitué, avec des composés de formule III, 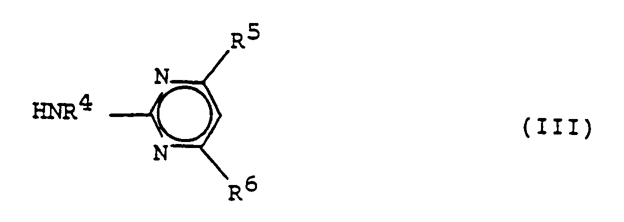
dans laquelle
R⁴, R⁵ et R⁶ sont définis comme ci-dessus,
dans un solvant organique inerte pour donner les composés de formule I.

2. Procédé selon la revendication 1, caractérisé en ce que, R¹, R² représentent, indépendamment l'un de l'autre, un groupe alkyle en C₁-C₃ ou alcényle en C₁-C₃, R³ et R⁴ un atome d'hydrogène, R⁵ et R⁶ indépendamment l'un de l'autre un groupe alkyle en C₁-C₂ ou alkoxy en C₁-C₂.

3. Procédé selon la revendication 1 ou 2, caractérisé en ce que, R¹ représente un groupe alkyle en C₁-C₂, R² un groupe alkyle en C₁-C₃ ou alcényle en C₁-C₃, R³ et R⁴ un atome d'hydrogène, R⁵ un groupe méthyle ou méthoxy et R⁶ un groupe méthyle ou méthoxy.

4. Procédé selon une ou plusieurs des revendication 1 à 3, caractérisé en ce que, R⁷ est un groupe alkyle en C₁-C₄, haloalkyle en C₁-C₄ ou phényle.

5. Procédé selon une ou plusieurs des revendication 1 à 4, caractérisé en ce que l'on utilise comme solvant organique inerte des hydrocarbures aromatiques, des hydrocarbures aromatiques halogénés ou des solvants polaires aprotiques ou des mélanges des solvants cités.

6. Procédé selon une ou plusieurs des revendication 1 à 5, caractérisé en ce qu'on le réalise à des températures de réaction de 0°C à la température d'ébullition du solvant utilisé.

7. Procédé selon la revendication 6, caractérisé en ce que la température de réaction est entre la température ambiante et 110°C.

8. Procédé selon une ou plusieurs des revendication 1 à 7, caractérisé en ce que l'on fait réagir les composés II et III dans le rapport molaire de 1:1 à 1,2:1.

9. Procédé selon la revendication 8, caractérisé en ce que le rapport molaire est de 1:1 à 1,1:1.

10. Procédé selon une ou plusieurs des revendication 1 à 9, caractérisé en ce que l'on réalise le procédé en discontinu ou en continu.
